(19) Europäisches Patentamt

European Patent Office

Office européen des brevets

(11) **EP 4 681 630 A1**

(12) **EUROPEAN PATENT APPLICATION**

(43) Date of publication:
**21.01.2026 Bulletin 2026/04**

(21) Application number: **25189490.3**

(22) Date of filing: **15.07.2025**

(51) International Patent Classification (IPC):
**A61B 5/11** *(2006.01)*

(52) Cooperative Patent Classification (CPC):
**A61B 5/0205; A61B 5/02438; A61B 5/1118**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **16.07.2024 US 202418774177
14.08.2024 US 202418804697**

(71) Applicant: **Medidata Solutions, Inc.
New York, NY 10014 (US)**

(72) Inventors:
• **ALPHONSE, Sebastian Anand
New York, NY 10014 (US)**
• **CERUOLO, Melissa
New York, NY 10014 (US)**

(74) Representative: **Fish & Richardson P.C.
Highlight Business Towers
Mies-van-der-Rohe-Straße 8
80807 München (DE)**

(54) **A MEDICAL MONITORING SYSTEM WITH CARDIAC AND ACCELERATION SENSORS**

(57) A medical monitoring system includes a sensor apparatus configured to be attached to a user's body and an electronic device communicatively coupled to the sensor apparatus. The sensor apparatus includes physiological sensor(s) configured to obtain physiological data representing a cardiac activity of a user and acceleration sensor(s) configured to obtain acceleration data representing a movement of the user. The electronic device includes processor(s) configured to: (i) cause the sensor apparatus to obtain the physiological data and the acceleration data during a time period; (ii) determine, based on the physiological data and the acceleration data, a biomarker representing a health of the user, where the biomarker represents a relationship between a speed of the movement of the user and the cardiac activity of the user during the time period; (iii) generate a data structure representing the biomarker, and (iv) store the data structure in a hardware storage device.

FIG. 1

**Description**

CROSS REFERENCE TO RELATED APPLICATIONS

The present application claims priority to United States Patent Application No. 18/774,177, filed on July 16, 2024, the entire contents of which are incorporated herein for all purposes by this reference.

TECHNICAL FIELD

This description generally relates to systems and methods for monitoring a user's medical condition using physiological sensors.

BACKGROUND

In general, a user's health can be assessed by measuring one or more physiological characteristics of the user, and comparing the measured physiological characteristics to a health reference. For example, a user having physiological characteristics that meet or exceed a particular health reference may be in good health, whereas a user having physiological characteristics that do not meet the health reference may be in poor health.

SUMMARY

In general, a medical monitoring system can be used to monitor a user's health and to facilitate treatment of the user.

In an example implementation, a medical monitoring system includes a sensor apparatus configured to obtain sensor data representing one or more physiological characteristics of a user, and one or more processor modules (e.g., computer processors) configured to process the sensor data and determine one or more biomarkers representing the user's medical condition.

For instance, the medical monitoring system can obtain sensor data while the user is exerting physical effort, such as while the user is continuously walking for a period of time. The sensor data can include measurements of the user's cardiac activity, such as the user's heartbeats during the period of time. Further, the sensor data can include measurements of the user's movements (e.g., acceleration data obtained using one or more acceleration sensors) during the period of time. Based on these measurements, the medical monitoring system can determine a biomarker that represents the user's physical health, such as the user's functional capacity and/or cardiopulmonary condition. Further, the medical monitoring system can present the biomarker to the user or another user (e.g., a health care provider) to facilitate treatment of the user and/or monitoring of the user's health over time.

In some implementations, the biomarker can be determined, at least in part, by (i) determining a median of interbeat intervals (IBI) of user's heartbeats over the period of time and an acceleration data of a movement of the user over that period of time, and (ii) combining the median of IBIs and the acceleration data. For example, combining the median of IBIs and the acceleration data can include multiplying the median of IBIs and the acceleration data. Further, the value of the biomarker can represent the relative health of the user. For example, over a particular range of values of the biomarker, a higher value can indicate that the user health is relatively high, whereas a lower value for the biomarker can indicate that the user health is relatively low.

Further, in at least some implementations, the biomarker obtained by the medical monitoring system can be used as an estimate or approximation of other biomarkers that represent a user's health that would otherwise require manual input from the user or third-party observer to calculate.

For example, in a traditional "six-minute walk test" (6MWT), a user walks continuously during a six minute period of time. Upon the completion of the period of time, an observer (e.g., a health care provider in a clinical setting) measures the distance that the user has walked during the period of time, and uses the measured distance as a biomarker representing the user's health. For example, if the user walked a long distance during the period of time, the biomarker is greater in value and represents that the user's health is relatively high. If the user walked a short distance during the period of time, the biomarker is lower in value and represents that the user's health is relatively low.

However, the traditional "six-minute walk test" may be difficult to perform in practice, and may be subject to variability. For example, a trained observer may not be readily available to conduct the test. In the absence of a trained observer, the user may conduct the test themselves incorrectly (e.g., measure the distance incorrectly, walk for the incorrect amount of time, etc.), which may negatively affect the accuracy and reliability of the results.

Further, the biomarker of the traditional "six-minute walk test" does not take into account or directly assess the user's physiological response (e.g., cardiovascular response, cardiac performance) to user's physical exertion (e.g., walking). Rather, as described above, the biomarker of the traditional "six-minute walk test" uses the measured distance during a certain set period of time as a biomarker representing the user's health.

In contrast, the biomarker obtained by the medical monitoring system described herein takes into account or directly assesses the user's physiological response to the user's physical exertion. For example, the biomarker takes into account a correlation between user's heartbeat and walking speed by combining cardiac data and acceleration data of the user. For example, the biomarker can represent the walking speed per the heartbeat of the user. Further, the biomarker obtained by the medical monitoring system does not require a trained observer or manual input from the user themselves. Accordingly, the user can use the medical monitoring system to monitor their health more easily and accurately (e.g., in home or outside environment, without the supervision of a health care provider).

The implementations described herein can provide various technical benefits. As an example, the implementations described herein allow a computer system to automatically determine a user's health based on sensor data (e.g., the cardiac data, the acceleration data, etc.), without requiring manual feedback from a human. Further, the medical monitoring system can provide this functionality by performing computer-specific operations on input data in an objective manner (and in a manner that is not feasible for a human to perform), rather than relying on a human interpretation of the input data which may be less suitable for performance by a computer. Further, the biomarker that is derived or determined from the implementations can be used to determine the user's health by directly assessing the user's physiological response to the user's physical exertion.

As another example, the implementations described herein can be used to facilitate monitoring and treatment of a user, thereby improving the user's health. For example, a medical monitoring system can generate a biomarker representing the user's health, and provide the biomarker to the user or another user (e.g., a health care provider). Based on this information, the user or other user can take active steps to improve the user's health, such as conducting further tests to diagnose the user's medical condition, changing the user's behavior, lifestyle, and/or diet, or any other action.

In an aspect, a method includes using an electronic device to (i) cause one or more physiological sensors of a sensor apparatus to obtain physiological data representing a cardiac activity of a user during a time period; (ii) cause one or more acceleration sensors of the sensor apparatus to obtain acceleration data representing a movement of the user during the time period; (iii) determine, based on the physiological data and the acceleration data, a biomarker representing a health of the user, where the biomarker represents a relationship between a speed of the movement of the user and the cardiac activity of the user during the time period; (iv) generate a data structure representing the biomarker; and (v) store the data structure in a hardware storage device.

In some implementations, determining the biomarker based on the physiological data and the acceleration data includes determining, based on the physiological data, IBIs of a heart rate of the user during the time period and determining a median IBI among the IBIs during the time period.

In some implementations, the one or more physiological sensors includes at least one of a photoplethysmography (PPG) sensor or an electrocardiogram (ECG) sensor. For instance, the physiological data can be obtained from at least one of the PPG sensor or the ECG sensor. Further, for instance, the IBIs can be determined based on at least one of PPG peaks or R-peaks of the physiological data.

In some implementations, determining the biomarker based on the physiological data and the acceleration data includes multiplying the median IBI by the acceleration data.

In some implementations, generating the data structure includes combining the biomarker with other physiological data of the user. Moreover, in some implementations, the electronic device can be used to cause the biomarker to be presented to the user. For instance, causing the biomarker to be presented to the user includes outputting the data structure on a display of a user interface.

Other embodiments of this aspect include corresponding computer systems, apparatus, and computer programs recorded on one or more computer storage devices, each configured to perform the actions or operations described herein. A system of one or more computers can be configured to perform particular actions by virtue of having software, firmware, hardware, or a combination of them installed on the system that in operation causes or cause the system to perform the actions. One or more computer programs can be configured to perform particular actions by virtue of including instructions that, when executed by a data processing apparatus, cause the apparatus to perform the actions.

The details of one or more embodiments of the subject matter of this specification are set forth in the accompanying drawings and the description below. Other features, aspects, and advantages of the subject matter will become apparent from the description, the drawings, and the claims.

BRIEF DESCRIPTION OF THE DRAWINGS

FIG. 1 is a diagram of an example medical monitoring system.
FIGS. 2A and 2B are diagrams of example operations of a medical monitoring system.
FIGS. 3 and 4 are flow chart diagrams of example processes for monitoring a user's health.

FIG. 5 is a diagram of an example computer system.

Like reference numbers and designations in the various drawings indicate like elements.

DETAILED DESCRIPTION

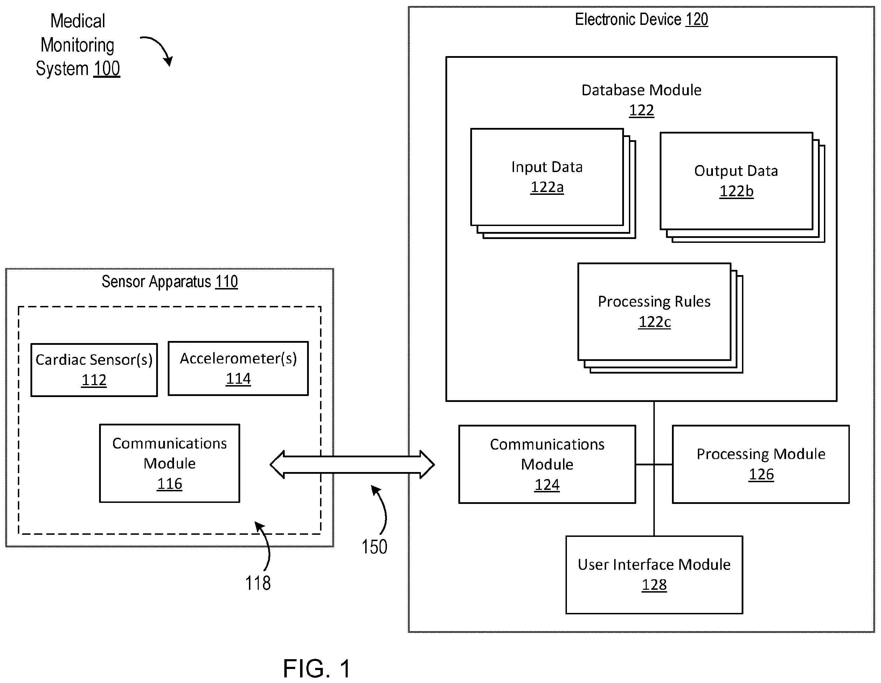

FIG. 1 shows an example medical monitoring system 100 for automatically monitoring the health of a user. The medical monitoring system 100 includes a sensor apparatus 110 and an electronic device 120 that are communicatively coupled to one another (e.g., via one or more wired or wireless communications links 150). In general, the medical monitoring system 100 obtains sensor data regarding a user using the sensor apparatus 110, and processes the sensor data using the electronic device 120 to determine one or more biomarkers representing the user's medical condition.

The sensor apparatus 110 includes one or more sensors configured to obtain measurements regarding a physiology of the user, a behavior of the user, and/or any other characteristics of the user.

For example, as shown in FIG. 1, the sensor apparatus 110 can include one or more cardiac sensors 112 configured to obtain sensor data representing the cardiac activity of a user. In some implementations, the cardiac sensor(s) 112 can include or more ECG sensors and/or one or more PPG sensors. In some implementations, the cardiac sensors 112 can obtain sensor data at 250 Hz (or approximately 250 Hz).

Further, the sensor apparatus 110 can include one or more accelerometers 114 configured to obtain sensor data representing a movement or motion of the user in one or more directions. For example, at least some of the accelerometers 114 can be tri-axis accelerometers that are configured to measure acceleration in three directions (e.g., the x-direction, the y-direction, and the z-direction on a Cartesian coordinate system). In some implementations, the accelerometers 114 can obtain sensor data at 32 Hz (or approximately 32 Hz).

Further, the sensor apparatus 110 includes a communications module 116 configured to transmit data and/or receive data from the electronic device 120. As an example, the communications module 116 can include one or more receivers, transmitters, and/or transceivers. In some implementations, the communications module 116 can communicate with the electronic device 120 via one or more wireless links (e.g., serial links, Ethernet links, etc.) and/or wireless links (e.g., Wi-Fi links, Bluetooth links, etc.).

Further, the sensor apparatus 110 is configured to be worn by a user. For example, as shown in FIG. 1, the sensor apparatus 110 can include a substrate 118 that is configured to be affixed to the user's body (e.g., via an adhesive). Further, the cardiac sensors 112, accelerometer 114, and communications module 116 can be attached to the substrate, such that they are secured to the user's body. In some implementations, the at least a portion of the substrate 118 can be composed of compliant materials such as silicone, rubber, elastomers, and/or any other flexible material. In some implementations, the at least a portion of the compliant substrate 118 can be composed of rigid materials such as rigid metals, rigid plastics, etc. In some implementations, the sensor apparatus 110 can be worn by the user without the substrate 118. For example, the sensor apparatus 110 can be disposed on the user's body through a wearable accessory or item (e.g., wristband).

In general, the electronic device 120 is configured to receive sensor data obtained by the sensor apparatus 110, and process the sensor data to determine one or more biomarkers representing the user's medical condition. Further, the electronic device 120 is configured to present information regarding the biomarkers and any other information to the user and/or another user (e.g., a health care provider).

In general, the electronic device 120 can include any number of devices that are configured to receive, process, and transmit data. Examples of the electronic device 120 include client computing devices (e.g., desktop computers or notebook computers), server computing devices (e.g., server computers or cloud computing systems), mobile computing devices (e.g., cellular phones, smartphones, tablets, personal data assistants, notebook computers with networking capability), wearable computing devices (e.g., smart phones or headsets), and other computing devices capable of receiving, processing, and transmitting data. In some implementations, the electronic device 120 can include computing devices that operate using one or more operating systems (e.g., Microsoft Windows, Apple macOS, Linux, Unix, Google Android, and Apple iOS, among others) and one or more architectures (e.g., x86, PowerPC, and ARM, among others).

In FIG. 1, the electronic device 120 is illustrated as a single component. However, in practice, the electronic device 120 can be implemented on one or more computing devices (e.g., each computing device including at least one processor such as a microprocessor or microcontroller). As an example, the electronic device 120 can be a single computing device, such as a single smartphone. As another example, the electronic device 120 can include multiple computing devices that are connected via a network (e.g., the Internet, local area network etc.), and the components of the electronic device 120 can be maintained and operated on some or all of the computing devices. For instance, electronic device 120 can include several computing devices, and the components of the electronic device 120 can be distributed on one or more of these computing devices.

Moreover, the electronic device 120 is illustrated as a component that is separate component from the sensor apparatus 110. However, while the electronic device 120 can be a separate component from the sensor apparatus 110, the electronic device 120 can also include, be coupled with, or be adjacent to (e.g., in a housing) the sensor apparatus 110. For example, the electronic device 120 can be a wearable device that includes, is coupled with, or is adjacent to the sensor apparatus 110.

As shown in FIG. 1, the electronic device 120 includes a database module 122, a communications module 124, a processing module 126, and a user interface module 128. The operation modules can be provided as one or more computer executable software modules, hardware modules, or a combination thereof. For example, one or more of the operation modules can be implemented as blocks of software code with instructions that cause one or more processors to execute operations described herein. In addition, or alternatively, one or more of the operations modules can be implemented in electronic circuitry such as, e.g., programmable logic circuits, field programmable logic arrays (FPGA), or application specific integrated circuits (ASIC).

The communications module 124 is configured to transmit data and/or receive data from the sensor apparatus 110. As an example, the communications module 124 can include one or more receivers, transmitters, and/or transceivers. In some implementations, the communications module 124 can communicate with the sensor apparatus 110 (e.g., via the communication module 116) via one or more wired links (e.g., serial links, Ethernet links, etc.) and/or wireless links (e.g., Wi-Fi links, Bluetooth links, etc.).

The database module 122 maintains information related to the operation of the medical monitoring system 100.

As an example, the database module 122 can store input data 122a that is used as an input for determining one or more biomarkers representing a health of a user. For instance, the input data 122a can include at least some of the sensor data generated by the sensor apparatus 110 (e.g., cardiac data, acceleration data, etc.).

As another example, the database module 122 can store output data 122b generated by electronic device 120. As an example, the output data 122b can include one or more metrics or biomarkers generated by the electronic device 120 based on the input data 122a.

Further, the database module 122 can store processing rules 122c specifying how data in the database module 122 can be processed to perform the operations described herein.

As an example, the processing rules 122c can include one or more rules that specify how the input data 122a is formatted, parsed, and processed to determine one or more corresponding metrics or biomarkers regarding a user.

As another example, the processing rules 122c can include one or more rules that specify the conditions in which data is presented to a user (e.g., using the user interface module 128), and the manner in which the data is presented.

As another example, the processing rules 122c can include one or more rules that specify the manner in which data is stored for future retrieval and/or processing (e.g., using the database module 122).

Example data processing techniques are described in further detail below.

The processing module 126 processes data stored or otherwise accessible to the electronic device 120. For instance, the processing module 126 can be used to execute one or more of the operations described herein (e.g., by executing the processing rules 122c with respect to the input data 112a in order to generate the output data 122b).

The user interface module 128 is configured to present information to a user and/or to receive inputs from a user. As an example, the user interface module 128 can include one more display devices (e.g., display screens, touch screens, etc.) that are configured to present a user interface (e.g., graphical user interface, GUI) that enables users to interact with the electronic device 120 and/or the sensor apparatus 110. Example interactions include viewing data, transmitting data from one component to another, and/or issuing commands to the electronic device 120 and/or sensor apparatus 110. Commands can include, for example, any user instruction to one or more of the electronic device 120 and/or sensor apparatus 110 to perform particular operations or tasks. In some implementations, the user interface module can also present information to a user aurally (e.g., using one or more speakers) and/or via haptic feedback (e.g., using one more haptic generators, such as a vibration generation).

In some implementations, a software application can be used to facilitate performance of the tasks described herein. As an example, an application can be installed on the electronic device 120. Further, a user can interact with the application to input data and/or commands to the electronic device 120, and review data generated by the call electronic device 120.

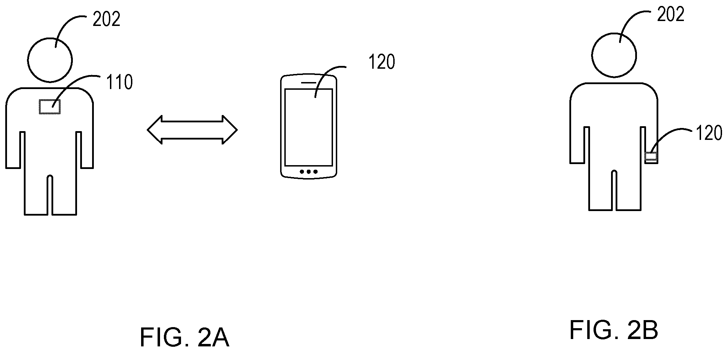

FIGS. 2A and 2B illustrates an example implementation and operation of the medical monitoring system 100.

Referring to FIG. 2A, the sensor apparatus 110 is worn by a user 202 (e.g., affixed to the user's skin, such as on the user's chest). Further, the user 202 continuously walks for a period of time. For example, as the user 202 is walking, the sensor apparatus 110 obtains sensor data regarding the user 202 (e.g., cardiac data, acceleration data, etc.) at certain time interval (e.g., 10 seconds, 30 seconds, 1 minute, etc.), and transmits at least some of the sensor data to the electronic device 120 for further processing. For example, the sensor data can be obtained at consistent, predefined time intervals, such as every 10 seconds, every 30 seconds, every minute, etc. On the other hand, for example, the sensor data can be obtained at variable time intervals, where the sensor data can be more frequently obtained during certain periods and less so during others.

The electronic device 120 receives the sensor data, and processes the sensor data to determine a biomarker that represents the user's health. For example, the electronic device 120 can determine a biomarker that represents the user's functional capacity (e.g., the user's ability to function in a variety of circumstances, such as the user's ability to pertain physical activities) and/or cardiopulmonary condition (e.g., the health of the user's heart, lungs, circulatory system, etc.).

Further, the electronic device 120 can present the biomarker to the user 202 or another user (e.g., a health care provider) to facilitate treatment of the user 202 and/or monitoring of the user's health over time.

In some implementations, referring to FIG. 2B, the electronic device 120 can be a wearable wristband (e.g., a wristwatch, smart watch, etc.) that includes, coupled with, or be adjacent to (e.g., in the same housing) the sensor apparatus 110. For example, the electronic device 120 can be disposed at the user's wrist and the sensor apparatus 110 can measure the sensor data regarding the user 202 that include cardiac data (e.g., PPG data, ECG data) and the acceleration data.

In some implementations, the medical monitoring system 100 can instruct the user regarding how to conduct a test using the medical monitoring system 100. For example, the medical monitoring system 100 can instruct the user (e.g., using a display screen, audio speaker, etc.) to place the sensor apparatus 110 on their body and/or select a certain time duration (e.g., the period of time or time interval for which one set of sensor data will be obtained to determine the biomarker). As described above, the medical monitoring system 100 can determine the biomarker based on the sensor data.

In some implementations, the medical monitoring system 100 can obtain the sensor data without requiring that the user enter a specific period of time. For example, the medical monitoring system 100 may have default time duration entered for which one set of sensor data will be obtained to determine the biomarker. Moreover, for example, multiple sets of sensor data can be obtained based on multiple periods of time duration to determine the biomarker. Moreover, for example, each biomarker determined for each set of sensor data can be averaged to determine the biomarker for multiple sets of sensor data. Moreover, in some implementations, the medical monitoring system 100 can continuously obtain sensor data regarding the user (e.g., during the course of the day), and automatically select a period of time for which to determine the biomarker. For example, the medical monitoring system 100 can determine, based on the sensor data, that the user has been continuously walking during a particular period of time. The medical monitoring system 100 can select sensor data from that period of time, and determine the biomarker based on the selected sensor data. This can be beneficial, for example, as it allows the medical monitoring system 100 to unobtrusively monitor the user's health over time (e.g., without requiring that the user manually initiate a test and/or without requiring that the user perform a prescribed physical activity).

In some implementations, the biomarker can be determined, at least in part, by (i) determining a median IBI among IBIs of user's heartbeats over the period of time (e.g., time interval) and acceleration data of a movement of the user over that period of time, and (ii) combining the median IBI and the acceleration data.

For example, user's cardiac data can be obtained over a span of one-minute (or every one-minute interval) using the sensor apparatus 110, and based on such cardiac data, R-peaks (e.g., for ECG data) or systolic peaks (e.g., for PPG data) can be extracted using the electronic device 120. Based on the R-peaks or the systolic peaks, number of IBIs, as well as the median IBI among the IBIs, can be determined using the electronic device 120. In some implementations, user's cardiac data obtained from the sensor apparatus 110 can include the extracted R-peaks or systolic peaks.

Moreover, for example, combining the median IBI and the acceleration data can include multiplying the median IBI and the acceleration data. As an example, the biomarker X can be determined using the relationship:

$$X = median\ IBI * acceleration \qquad \text{(Eq. 1)},$$

Where *median IBI* is a median of multiple IBIs of heartbeats measured during the period of time or time interval and *acceleration* represents a magnitude of acceleration vector in in x, y, z directions. For example, *acceleration* can be determined by squaring x, y, and z components of acceleration vector, and then taking the square root of their sum. Moreover, for example, *median IBI* can be represented in a unit of time (e.g., second(s), millisecond(s)) per heartbeat, and *acceleration* can be represented in a unit of average acceleration variation in the movements of the user over that period of distance (e.g., meter) per time squared (e.g., $second^2$). Moreover, for example, the biomarker X can be represented in speed per heartbeat (e.g., meter / (second * heartbeat)).

In some implementations, number of heartbeats that is used to determine the IBIs and the median IBI can be determined by segmenting the cardiac data (e.g., ECG data, PPG data, etc.) into several segments, where each segment corresponds to a different respective single heartbeat. For example, the cardiac data can be segmented by identifying features of the cardiac data (e.g., peaks, troughs, etc.) that are indicative of a heartbeat. Further, the number of segments can be counted to determine the total number of heartbeats.

Further, the value of the biomarker can represent the relative health of the user. For example, over a particular range of values of the biomarker, a higher value can indicate that the user health is relatively high, whereas a lower value for the biomarker can indicate that the user health is relatively low. For example, such biomarker can be beneficial and be used as an index for various purposes, in assessing the user's health quality, tracking disease progression, predicting an outcome of a clinical trial, determining health-related statistical data, etc.

As an illustrative example, during a period of time, a first user and a second user walk in a similar manner. The first user's biomarker during the period of time is greater than that of the second user, which may indicate that the first user's functional ability and/or cardiopulmonary health is better than that of the second user. This difference reflected in the biomarkers of the two users, in which the value of the first user's biomarker is greater than that of the second user.

As another example, for illustrative purposes, for a set of distance (e.g., 100 m), a first user and a second user walk at different speed or at the same speed. If the first user's biomarker during the period of time is greater than that of the second user, this may indicate that the first user's functional ability and/or cardiopulmonary health is better than that of the second user.

As another example, without being bounded by time constraints or distance, a user walks at certain terrain (e.g., flat terrain, boardwalk, walking path). If the user's biomarker is greater than a predetermined threshold or an average biomarker among multiple biomarker data of different people at similar terrain, this can indicate that the user's functional ability and/or cardiopulmonary health is better than that of an average person or average value among multiple biomarker data of different people in a sample data set. Further, the user's biomarker can vary among different environmental conditions or controlled conditions (e.g., inclined surface, climbing, specific activity) and the user's biomarker can be compared to an average or pre-determined values that correspond to these corresponding environmental conditions. In some implementations, the user can communicate with (e.g., manually input through touch or voice) the electronic device 120 to input the environmental condition or controlled condition prior to a walk or specific exercise and initiating heartbeat and acceleration data measurement.

In some implementations, the medical monitoring system 100 can present value of the biomarker. For example, the value of the biomarker can be presented as a graphical display element on a GUI (e.g., as presented on a display device) and/or as an audio notification (e.g., as presented using an audio speaker).

In some implementations, the medical monitoring system 100 can determine a descriptive health category of the user based on the value of the biomarker. For example, medical monitoring system 100 can access several health categories (e.g., "good," "fair," "poor," etc.), each associated with a different respective range of values. The medical monitoring system 100 can determine that the value of the biomarker falls within a particular one of the ranges, and determine that the health of the user corresponds to the category associated with that range. This can be beneficial, for example, as a descriptive health category may be easier for a user to understand, compared to the specific value of biomarker.

In some implementations, the medical monitoring system 100 can also determine additional information regarding the physiology of the user. For example, based on the cardiac data, the medical monitoring system 100 can determine cardiac metrics such as the user's resting and peak heart rates, and heart rate recovery after the walk. As another example, based on the acceleration data, the medical monitoring system 100 can determine gait metrics regarding the user's walking patterns. This additional information also can be presented to the user and/or another user (e.g., graphically, aurally, haptically, etc.).

In some implementations, the medical monitoring system 100 can be used to facilitate treatment of user 202, thereby improving the user's health. For example, the medical monitoring system 100 can generate and provide the biomarker representing the user's health to the user 202 or another user (e.g., a health care provider). Based on this information, the user 202 or other user can take active steps to improve the user's health, such as conducting further tests to diagnose the user's medical condition, changing the user's behavior, lifestyle, and/or diet, or any other action.

In some implementations, medical monitoring system 100 can determine one or more recommendations for improving a health of the user based on the biomarker, and cause the recommendations to be presented to the user.

In some implementations, the medical monitoring system 100 can also provide instructions to the user 202 regarding how to operate the medical monitoring system 100. For example, the medical monitoring system 100 (e.g., using the electronic device 120) can provide instructions to the user 202 to affix the sensor apparatus 110 to a particular location on the user's body (e.g., the user's chest), and to walk back and forth between two points for a particular length of time (e.g., six minutes). Further, upon completion of the user's walk, the medical monitoring system 100 can provide feedback to the user 202 regarding the sensor data that was obtained and the biomarker that was generated based on the sensor data. This can be beneficial, for example, in aiding the user 202 to conduct a test of their physical health, even if the user 202 does not have any prior experience with conducting the test. In some implementations, the instructions can be provided by the medical monitoring system 100 graphically (e.g., using one or more display screens and/or aurally (e.g., using one or more audio speakers).

*Example Processes*

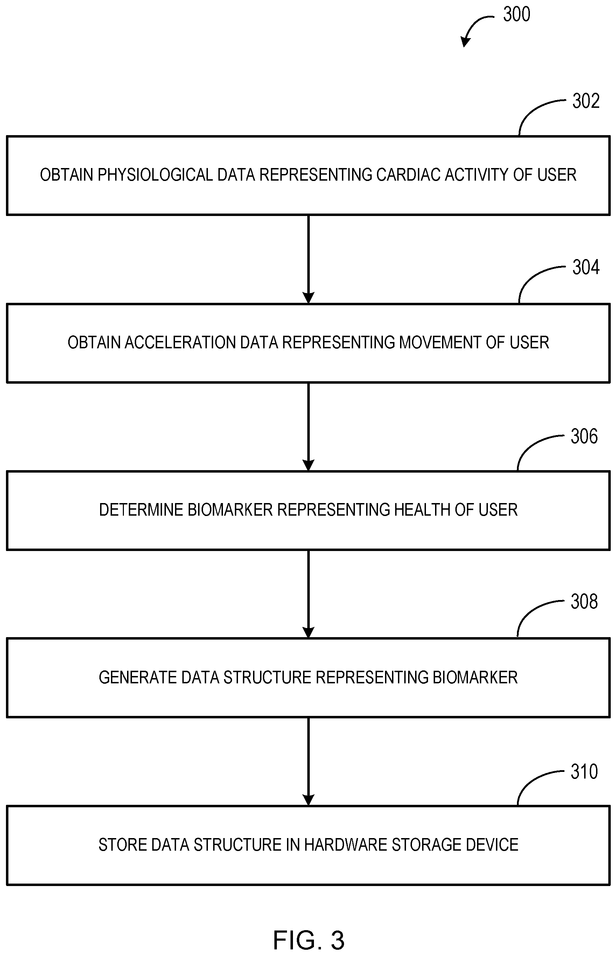

FIG. 3 is a flow chart diagram of an example process 300 for monitoring a user's health using physiological sensors. The process 300 can be implemented by a processor-based system, such as the medical monitoring system 100 and a system 500, described in this disclosure.

At 302, physiological data representing a cardiac activity of user is obtained. For example, while the user is walking or moving, the physiological data can be obtained by one or more cardiac sensors (e.g., the cardiac sensor(s) 112).

For example, a processor-based electronic device (e.g., the electronic device 120) which is in communication (e.g., wired or wireless communication) with the one or more cardiac sensors, can be used to cause the one or more cardiac sensors to obtain the physiological data. In some implementations, the electronic device includes, is coupled with, or is adjacent to (e.g., in the same housing) the one or more cardiac sensors.

The physiological data can include or correspond to ECG data or PPG data. For example, the ECG data includes ECG waveform including various peaks and troughs that reflect different phases of cardiac cycle. In particular, the ECG data includes QRS complex, and R peak of the QRS complex can be used for determining heart rate of the user. Moreover, for example, the PPG data includes PPG waveform including various peaks and troughs that reflect different phases of cardiac cycle. In particular, the PPG data includes systolic peak, which can be used for determining the heart rate of the user.

The physiological data can be obtained at certain time interval (e.g., 10 seconds, 30 seconds, 1 minute, etc.), and be transmitted to the electronic device for further processing. In some implementations, the physiological data can be obtained at consistent, predefined time intervals, such as every 10 seconds, every 30 seconds, every minute, etc. In some implementations, the physiological data can be obtained at variable time intervals, where the physiological data can be more frequently obtained during certain periods and less so during others. After the physiological data is obtained, the physiological data are transmitted to the electronic device for further processing.

For example, after or in response to receiving the physiological data, the electronic device can extract R-peaks (in case the physiological data includes or corresponds to ECG data). Further, based on these extracted R-peaks, the electronic device can determine the interbeat intervals (IBI) of user's heartbeats during the time interval. In some implementations, the R-peaks and/or the IBIs can be extracted from the one or more cardiac sensors and the electronic device can receive the extracted R-peaks and/or the IBIs of the user. In some implementations, in case the physiological data includes or corresponds to PPG data, the electronic device can extract PPG peaks or systolic peak of the PPG peaks after or in response to receiving the physiological data. Further, based on these extracted PPG peaks or systolic peaks of the PPG peaks, the electronic device can determine the IBIs of the user's heartbeats during the time interval. In some implementations, the systolic peaks and/or IBIs can be extracted from the one or more cardiac sensors and the electronic device can receive the extracted systolic peaks and/or the IBIs of the user.

For example, a processor of the electronic device (e.g., the one or more processors described in FIG. 1) can be configured to track the physiological data obtained at the predefined time intervals and determine the R-peaks and/or the IBIs over the one or more of the predefined time intervals.

At 304, acceleration data representing movement of user is obtained. For example, while the user is walking or moving, the acceleration data can be obtained by one or more accelerometers (e.g., the accelerometer(s) 114). For example, the electronic device that is in communication (e.g., wired or wireless communication) with the one or more accelerometers can be used to cause the one or more accelerometers to obtain the acceleration data. In some implementations, the electronic device includes, is coupled with, or is adjacent to (e.g., in the same housing) the one or more accelerometers.

For example, the acceleration data can include or represent accelerations or acceleration vectors of the user in three directions (e.g., the x-direction, the y-direction, and the z-direction on a Cartesian coordinate system) while the user is walking or moving. For example, in a similar manner with respect to the physiological data, the acceleration data can be obtained at certain time interval (e.g., 10 seconds, 30 seconds, 1 minute, etc.), and be transmitted to the electronic device for further processing. In some implementations, the acceleration data can be obtained at consistent, predefined time intervals, such as every 10 seconds, every 30 seconds, every minute, etc. In some implementations, the acceleration data can be obtained at variable time intervals, where the acceleration data can be more frequently obtained during certain periods and less so during others. After the physiological data is obtained, the physiological data are transmitted to the electronic device for further processing.

For example, after or in response to receiving the acceleration data, the electronic device can determine an acceleration of the user by squaring x, y, and z components of acceleration data or vector, and then taking the square root of their sum. In some implementations, the acceleration of the user can be determined at the one or more accelerometers and the electronic device can receive the data representing the acceleration of the user.

In some implementations, after or in response to receiving the acceleration data and prior to squaring the x, y, and z components of the acceleration data or vector, the electronic device can apply band-pass filter to the received acceleration data or vector for each axis (e.g., x, y, z axis) independently to eliminate bias and isolate the acceleration component. After filtering, the x, y, and z components, which correspond to the band-pass filtered acceleration data for the x, y, and z axes, respectively, can be squared, summed, and then the square root of their sum can be taken to determine the acceleration of the user.

At 306, biomarker representing the health of user is determined. In particular, the biomarker can be determined based on the physiological data and the acceleration data. For example, determining the biomarker can include determining, based on the physiological data and by the electronic device, the IBIs of the heart rate of the user during a time period (e.g., the time interval), and determining a median IBI among the plurality of IBIs during the time period. For example, the IBIs are determined based on the extracted R-peaks of the physiological data (in case the physiological data includes or corresponds to the ECG data). In another example, the IBIs are determined based on the PPG peaks or extracted systolic peak of the physiological data (in case the physiological data includes or corresponds to the PPG data).

Further, determining the biomarker can include multiplying the median IBI by the acceleration data. For example, determining the biomarker can include multiplying the median IBI by the acceleration of the user. For example, the acceleration can correspond to an acceleration determined based on the application of band-pass filtering, as described above.

As described above with respect to the discussion of Equation 1, for example, the biomarker can be represented in units of speed per heartbeat (e.g., meter / (second * heartbeat)).

Further, as described above, the value of the biomarker can represent the relative health of the user. For example, over a particular range of values of the biomarker, a higher value can indicate that the user health is relatively high, whereas a lower value for the biomarker can indicate that the user health is relatively low.

In some implementations, multiple sets of sensor data (including the physiological data and the acceleration data) can be obtained to determine the biomarker. For example, the multiple sets of the sensor data can be obtained at multiple time intervals that amount up to, or form portions of, the period of time, and the biomarker for the period of time can be determined. For example, each biomarker determined for each set of sensor data for respective time interval can be averaged to determine the biomarker for multiple sets of the sensor data.

At 308, data structure representing the biomarker is generated. For example, the electronic device can format the data into a standardized data format, such as JavaScript Object Notation (JSON) or Extensible Markup Language (XML) format. The standardized data format can be other appropriate data format suitable for storage in a data store or a hardware storage device.

At 310, the data structure is stored in the hardware storage device. For example, the data structure can be stored in one or more storage devices (e.g., one or more storage devices 530, data store, etc.).

In some implementations, in supplant of, or in addition to, storing the data structure in the hardware storage device, the data structure may be output to a user interface (e.g. using the user interface module 128). For example, in cases of outputting the data structure for user display without storing the data structure, the data structure may be generated in other format appropriate for display at step 308. For example, in cases of outputting the data structure for user display after storing the data structure, the data structure can be converted from the storage format to the appropriate display format, and be output.

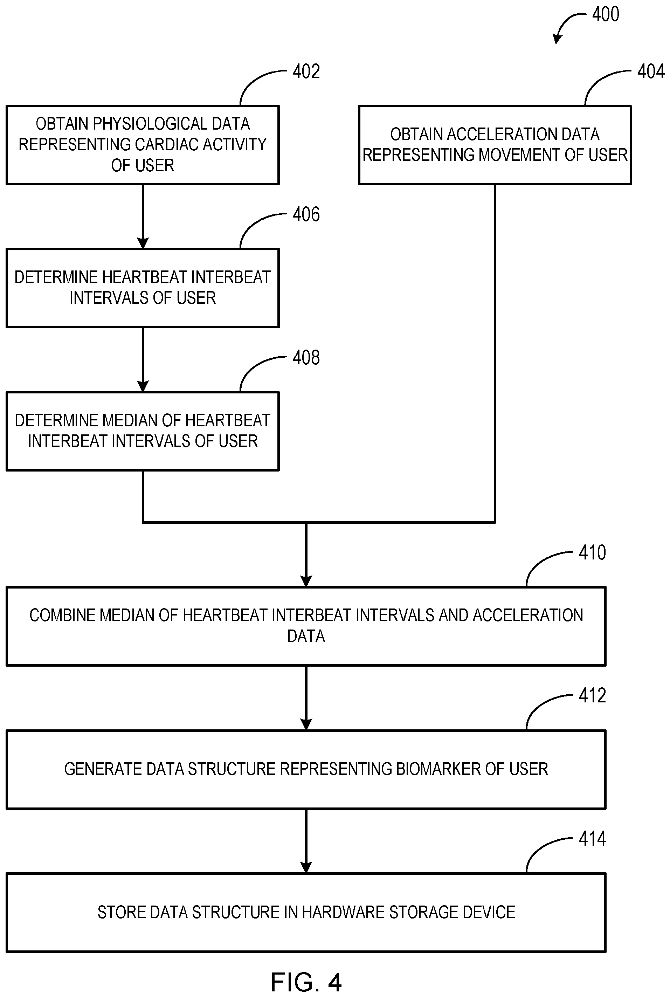

FIG. 4 is a flow chart diagram of an example process 400 for monitoring a user's health using physiological sensors. The process 400 can be implemented by a processor-based system, such as the medical monitoring system 100 and a system 500, and be viewed in conjunction with the process 300.

At 402, physiological data representing cardiac activity of user is obtained. For example, while the user is walking or moving, the physiological data can be obtained by one or more cardiac sensors (e.g., the cardiac sensor(s) 112). As the technique used in step 402 can be the same as the technique used in step 302 of FIG. 3, the technique is not repeated here.

At 404, acceleration data representing a movement of the user can be obtained. For example, while the user is walking or moving, the acceleration data can be obtained by one or more accelerometers (e.g., the accelerometer(s) 114). As the technique used in step 404 can be the same as the technique used in step 304 of FIG. 3, the technique is not repeated here.

At 406, IBIs of heartbeat of the user are determined. For example, based on the physiological data (e.g., ECG data, PPG data) that includes waveform including various peaks and troughs that reflect different phases of cardiac cycle, R-peaks (for ECG data) or systolic peaks (for PPG data) are extracted, by an electronic device (e.g., the electronic device 120). For example, based on the extracted R-peaks or the systolic peaks, the IBIs of the heartbeat of the user for a certain time period are determined.

At 408, a median IBI among the determined (or extracted) IBIs is determined by the electronic device. In some implementations, in supplant of the median IBI, an average IBI of the IBIs can be determined.

At 410, the median IBI and the acceleration data are combined to determine the biomarker. For example, combining the median IBI and the acceleration data can include multiplying the median IBI and an acceleration of the acceleration data. For example, as described above with respect to the Equation 1, the acceleration can be determined by squaring $x$, $y$, and $z$ components of acceleration vector, and then taking the square root of their sum. Moreover, for example, median IBI can be represented in a unit of time (e.g., second(s), millisecond(s)) per heartbeat, and acceleration can be represented in a unit of average acceleration variation in the movements of the user over that period of distance (e.g., meter) per time squared (e.g., second$^2$). Moreover, for example, the biomarker can be represented in speed per heartbeat (e.g., meter / (second * heartbeat)).

At 412, data structure representing the biomarker is generated. For example, the electronic device can format the data into a standardized data format, such as JavaScript Object Notation (JSON) or Extensible Markup Language (XML) format. The standardized data format can be other appropriate data format suitable for storage in a data store.

At 414, the data structure is stored in a hardware storage device. For example, the data structure can be stored in one or more storage devices (e.g., one or more storage devices 530, data store, etc.). As the technique used in step 414 can be the same as the technique used in step 310 of FIG. 3, the technique is not repeated here.

*Example Computer Systems*

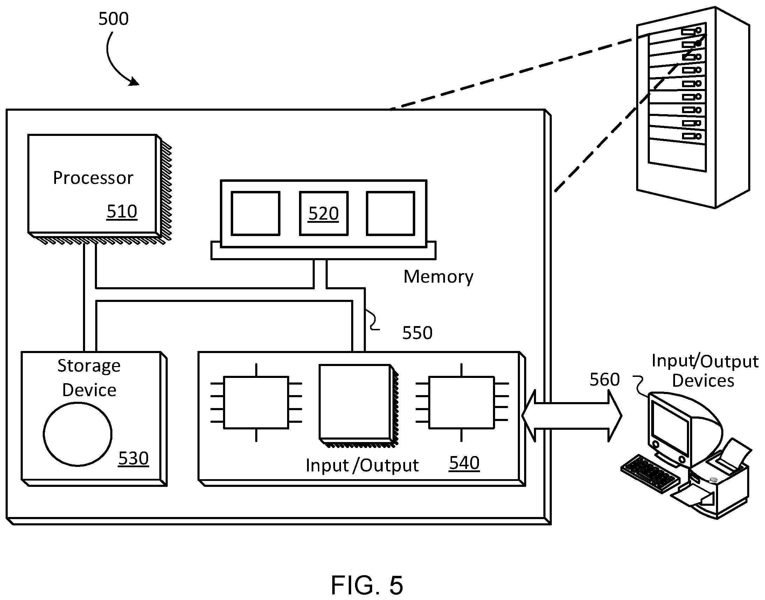

FIG. 5 depicts an example computing system, according to implementations of the present disclosure. The system 500 may be used for any of the operations described with respect to the various implementations discussed herein. The system 500 may be included in, used by, in communication with, or correspond to the electronic device 120. Further, the system 500 may include, used by, or in communication with the sensor apparatus 110. The system 500 may include one or more processors 510, a memory 520, one or more storage devices 530, and one or more input/output (I/O) devices 560 controllable through one or more I/O interfaces 540. The various components 510, 520, 530, 540, or 560 may be interconnected through at least one system bus 550, which may enable the transfer of data between the various modules and components of the system 500.

The processor(s) 510 may be configured to process instructions for execution within the system 500. The processor(s) 510 may include single-threaded processor(s), multi-threaded processor(s), or both. The processor(s) 510 may be configured to process instructions stored in the memory 520 or on the storage device(s) 530. The processor(s) 510 may include hardware-based processor(s) each including one or more cores. The processor(s) 510 may include general purpose processor(s), special purpose processor(s), or both.

The memory 520 may store information within the system 500. In some implementations, the memory 520 includes one or more computer-readable media. The memory 520 may include any number of volatile memory units, any number of non-volatile memory units, or both volatile and non-volatile memory units. The memory 520 may include read-only memory, random access memory, or both. In some examples, the memory 520 may be employed as active or physical memory by one or more executing software modules.

The storage device(s) 530 may be configured to provide (e.g., persistent) mass storage for the system 500. In some implementations, the storage device(s) 530 may include one or more computer-readable media. For example, the storage device(s) 530 may include a floppy disk device, a hard disk device, an optical disk device, or a tape device. The storage device(s) 530 may include read-only memory, random access memory, or both. The storage device(s) 530 may include one or more of an internal hard drive, an external hard drive, or a removable drive.

One or both of the memory 520 or the storage device(s) 530 may include one or more computer-readable storage media (CRSM). The CRSM may include one or more of an electronic storage medium, a magnetic storage medium, an optical storage medium, a magneto-optical storage medium, a quantum storage medium, a mechanical computer storage medium, and so forth. The CRSM may provide storage of computer-readable instructions describing data structures, processes, applications, programs, other modules, or other data for the operation of the system 500. In some implementations, the CRSM may include a data store that provides storage of computer-readable instructions or other information in a non-transitory format. The CRSM may be incorporated into the system 500 or may be external with respect to the system 500. The CRSM may include read-only memory, random access memory, or both. One or more CRSM suitable for tangibly embodying computer program instructions and data may include any type of non-volatile memory, including but not limited to: semiconductor memory devices, such as EPROM, EEPROM, and flash memory devices; magnetic disks such as internal hard disks and removable disks; magneto-optical disks; and CD-ROM and DVD-ROM disks. In some examples, the processor(s) 510 and the memory 520 may be supplemented by, or incorporated into, one or more application-specific integrated circuits (ASICs).

The system 500 may include one or more I/O devices 560. The I/O device(s) 560 may include one or more input devices such as a keyboard, a mouse, a pen, a game controller, a touch input device, an audio input device (e.g., a microphone), a gestural input device, a haptic input device, an image or video capture device (e.g., a camera), or other devices. In some examples, the I/O device(s) 560 may also include one or more output devices such as a display, LED(s), an audio output device (e.g., a speaker), a printer, a haptic output device, and so forth. The I/O device(s) 560 may be physically incorporated in one or more computing devices of the system 500, or may be external with respect to one or more computing devices of the system 500.

The system 500 may include one or more I/O interfaces 540 to enable components or modules of the system 500 to control, interface with, or otherwise communicate with the I/O device(s) 560. The I/O interface(s) 540 may enable information to be transferred in or out of the system 500, or between components of the system 500, through serial communication, parallel communication, or other types of communication. For example, the I/O interface(s) 540 may comply with a version of the RS-232 standard for serial ports, or with a version of the IEEE 1284 standard for parallel ports. As another example, the I/O interface(s) 540 may be configured to provide a connection over Universal Serial Bus (USB) or Ethernet. In some examples, the I/O interface(s) 540 may be configured to provide a serial connection that is compliant with a version of the IEEE 1394 standard.

The I/O interface(s) 540 may also include one or more network interfaces that enable communications between computing devices in the system 500, or between the system 500 and other network-connected computing systems. The network interface(s) may include one or more network interface controllers (NICs) or other types of transceiver devices configured to send and receive communications over one or more networks using any network protocol.

Computing devices of the system 500 may communicate with one another, or with other computing devices, using one or more networks. Such networks may include public networks such as the internet, private networks such as an institutional or personal intranet, or any combination of private and public networks. The networks may include any type of wired or wireless network, including but not limited to local area networks (LANs), wide area networks (WANs), wireless WANs (WWANs), wireless LANs (WLANs), mobile communications networks (e.g., 3G, 4G, Edge, etc.), and so forth. In some implementations, the communications between computing devices may be encrypted or otherwise secured. For example, communications may employ one or more public or private cryptographic keys, ciphers, digital certificates, or other credentials supported by a security protocol, such as any version of the Secure Sockets Layer (SSL) or the Transport Layer Security (TLS) protocol.

The system 500 may include any number of computing devices of any type. The computing device(s) may include, but are not limited to: a personal computer, a smartphone, a tablet computer, a wearable computer, an implanted computer, a mobile gaming device, an electronic book reader, an automotive computer, a desktop computer, a laptop computer, a notebook computer, a game console, a home entertainment device, a network computer, a server computer, a mainframe computer, a distributed computing device (e.g., a cloud computing device), a microcomputer, a system on a chip (SoC), a system in a package (SiP), and so forth. Although examples herein may describe computing device(s) as physical device(s), implementations are not so limited. In some examples, a computing device may include one or more of a virtual computing environment, a hypervisor, an emulation, or a virtual machine executing on one or more physical computing devices. In some examples, two or more computing devices may include a cluster, cloud, farm, or other grouping of multiple devices that coordinate operations to provide load balancing, failover support, parallel processing capabilities, shared storage resources, shared networking capabilities, or other aspects.

This specification uses the term "configured" in connection with systems and computer program components. For a system of one or more computers to be configured to perform particular operations or actions means that the system has installed on software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively, or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term "database" is used broadly to refer to any collection of data: the data does not need to be structured in any particular way, or structured at all, and it can be stored on storage devices in one or more locations. Thus, for example, the index database can include multiple collections of data, each of which may be organized and accessed differently.

Similarly, in this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable sub combination. Moreover, although features may be described above as acting in certain combinations and even initially be claimed as such, one or more features from a claimed combination can in some cases be excised from the combination, and the claimed combination may be directed to a sub combination or variation of a sub combination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

## EMBODIMENTS

Although the present invention is defined in the claims, it should be understood that the present invention can also (alternatively) be defined in accordance with the following embodiments:

1. A medical monitoring system comprising:

a sensor apparatus configured to be attached to a user's body, wherein the sensor apparatus comprises:

one or more physiological sensors configured to obtain physiological data representing a cardiac activity of a user, and
one or more acceleration sensors configured to obtain acceleration data representing a movement of the user; and

an electronic device communicatively coupled to the sensor apparatus, wherein the electronic device comprises one or more processors that are configured to:

cause the sensor apparatus to obtain the physiological data and the acceleration data during a time period,
determine, based on the physiological data and the acceleration data, a biomarker representing a health of the user, wherein the biomarker represents a relationship between a speed of the movement of the user and the cardiac activity of the user during the time period,
generate a data structure representing the biomarker, and
store the data structure in a hardware storage device.

2. The medical monitoring system of embodiment 1, wherein the biomarker represents a cardiopulmonary condition of the user.

3. The medical monitoring system of embodiment 1, wherein the biomarker is correlated with a walking speed of the user and the cardiac activity of the user.

4. The medical monitoring system of embodiment 1, wherein the one or more physiological sensors comprises an electrocardiogram (ECG) sensor.

5. The medical monitoring system of embodiment 1, wherein the one or more physiological sensors are configured to be secured to at least one of a user's chest or a user's wrist.

6. The medical monitoring system of embodiment 1, wherein the one or more physiological sensors comprises a photoplethysmography (PPG) sensor.

7. The medical monitoring system of embodiment 1, wherein the one or more acceleration sensors comprises a 3-axis accelerometer configured to measure an acceleration in three dimensions.

8. The medical monitoring system of embodiment 1, wherein determining the biomarker comprises:

determining, based on the physiological data, a plurality of interbeat intervals (IBI) of a heart rate of the user during the time period; and
determining a median IBI among the plurality of IBIs during the time period.

9. The medical monitoring system of embodiment 8, wherein the one or more physiological sensors comprises at least one of a photoplethysmography (PPG) sensor or an electrocardiogram (ECG) sensor, and
wherein the plurality of IBIs are determined based on at least one of PPG peaks or R-peaks of the physiological data.

10. The medical monitoring system of embodiment 8, wherein determining the biomarker comprises:
multiplying the median IBI by the acceleration data.

11. The medical monitoring system of embodiment 1, wherein the one or more processors are configured to:
cause the biomarker to be presented to the user.

12. The medical monitoring system of embodiment 11, wherein causing the biomarker to be presented to the user comprises:
outputting the data structure on a display of a user interface.

13. The medical monitoring system of embodiment 1, wherein generating the data structure comprises:
combining the biomarker with other physiological data of the user.

14. A method comprising:

causing, using an electronic device, one or more physiological sensors of a sensor apparatus to obtain physiological data representing a cardiac activity of a user during a time period;
causing, using the electronic device, one or more acceleration sensors of the sensor apparatus to obtain acceleration data representing a movement of the user during the time period;
determining, by the electronic device and based on the physiological data and the acceleration data, a biomarker representing a health of the user, wherein the biomarker represents a relationship between a speed of the movement of the user and the cardiac activity of the user during the time period;
generating, by the electronic device, a data structure representing the biomarker; and
storing, by the electronic device, the data structure in a hardware storage device.

15. The method of embodiment 14, wherein determining the biomarker based on the physiological data and the acceleration data comprises:

determining, based on the physiological data, a plurality of interbeat intervals (IBI) of a heart rate of the user during the time period; and
determining a median IBI among the plurality of IBIs during the time period.

16. The method of embodiment 15, wherein the one or more physiological sensors comprises at least one of a photoplethysmography (PPG) sensor or an electrocardiogram (ECG) sensor,

wherein the physiological data is obtained from at least one of the photoplethysmography (PPG) sensor or the electrocardiogram (ECG) sensor, and
wherein the plurality of IBIs are determined based on at least one of PPG peaks or R-peaks of the physiological data.

17. The method of embodiment 15, wherein determining the biomarker based on the physiological data and the acceleration data comprises:
multiplying the median **IBI** by the acceleration data.

18. The method of embodiment 14, wherein generating the data structure comprises:
combining the biomarker with other physiological data of the user.

19. The method of embodiment 14, further comprising:
causing, using the electronic device, the biomarker to be presented to the user.

20. The method of embodiment 19, wherein causing the biomarker to be presented to the user comprises:
outputting the data structure on a display of a user interface.

**Claims**

1.  A medical monitoring system comprising:

    a sensor apparatus configured to be attached to a user's body, wherein the sensor apparatus comprises:

    one or more physiological sensors configured to obtain physiological data representing a cardiac activity of a user, and
    one or more acceleration sensors configured to obtain acceleration data representing a movement of the user; and

    an electronic device communicatively coupled to the sensor apparatus, wherein the electronic device comprises one or more processors that are configured to:

    cause the sensor apparatus to obtain the physiological data and the acceleration data during a time period,
    determine, based on the physiological data and the acceleration data, a biomarker representing a health of the user, wherein the biomarker represents a relationship between a speed of the movement of the user and the cardiac activity of the user during the time period,
    generate a data structure representing the biomarker, and
    store the data structure in a hardware storage device.

2.  The medical monitoring system of claim 1, wherein the biomarker represents a cardiopulmonary condition of the user.

3.  The medical monitoring system of any one of claims 1 or 2, wherein the biomarker is correlated with a walking speed of the user and the cardiac activity of the user.

4.  The medical monitoring system of any one of claims 1 to 3, wherein the one or more physiological sensors comprise an electrocardiogram (ECG) sensor; and/or wherein the one or more physiological sensors are configured to be secured to at least one of a user's chest or a user's wrist; and/or wherein the one or more physiological sensors comprise a photoplethysmography (PPG) sensor; and/or wherein the one or more acceleration sensors comprise a 3-axis accelerometer configured to measure an acceleration in three dimensions.

5.  The medical monitoring system of any one of claims 1 to 4, wherein determining the biomarker comprises:

    determining, based on the physiological data, a plurality of interbeat intervals (IBI) of a heart rate of the user during the time period; and
    determining a median IBI among the plurality of IBIs during the time period.

6.  The medical monitoring system of claim 5, wherein the one or more physiological sensors comprises at least one of a photoplethysmography (PPG) sensor or an electrocardiogram (ECG) sensor, and

    wherein the plurality of IBIs are determined based on at least one of PPG peaks or R-peaks of the physiological data; and/or
    wherein determining the biomarker comprises:
    multiplying the median IBI by the acceleration data.

7.  The medical monitoring system of any one of claims 1 to 6, wherein the one or more processors are configured to:

    cause the biomarker to be presented to the user; optionally
    wherein causing the biomarker to be presented to the user comprises:
    outputting the data structure on a display of a user interface.

8.  The medical monitoring system of any one of claims 1 to 7, wherein generating the data structure comprises:
    combining the biomarker with other physiological data of the user.

9.  A method comprising:

    causing, using an electronic device, one or more physiological sensors of a sensor apparatus to obtain physiological data representing a cardiac activity of a user during a time period;

causing, using the electronic device, one or more acceleration sensors of the sensor apparatus to obtain acceleration data representing a movement of the user during the time period;

determining, by the electronic device and based on the physiological data and the acceleration data, a biomarker representing a health of the user, wherein the biomarker represents a relationship between a speed of the movement of the user and the cardiac activity of the user during the time period;

generating, by the electronic device, a data structure representing the biomarker; and

storing, by the electronic device, the data structure in a hardware storage device.

**10.** The method of claim 9, wherein determining the biomarker based on the physiological data and the acceleration data comprises:

determining, based on the physiological data, a plurality of interbeat intervals (IBI) of a heart rate of the user during the time period; and

determining a median IBI among the plurality of IBIs during the time period.

**11.** The method of claim 10, wherein the one or more physiological sensors comprises at least one of a photoplethysmography (PPG) sensor or an electrocardiogram (ECG) sensor,

wherein the physiological data is obtained from at least one of the photoplethysmography (PPG) sensor or the electrocardiogram (ECG) sensor, and

wherein the plurality of IBIs are determined based on at least one of PPG peaks or R-peaks of the physiological data.

**12.** The method of claim 10, wherein determining the biomarker based on the physiological data and the acceleration data comprises:

multiplying the median IBI by the acceleration data.

**13.** The method of any one of claims 9 to 12, wherein generating the data structure comprises:

combining the biomarker with other physiological data of the user.

**14.** The method of any one of claims 9 to 13, further comprising:

causing, using the electronic device, the biomarker to be presented to the user.

**15.** The method of claim 14, wherein causing the biomarker to be presented to the user comprises:

outputting the data structure on a display of a user interface.

FIG. 1

FIG. 2B

FIG. 2A

300

302

OBTAIN PHYSIOLOGICAL DATA REPRESENTING CARDIAC ACTIVITY OF USER

304

OBTAIN ACCELERATION DATA REPRESENTING MOVEMENT OF USER

306

DETERMINE BIOMARKER REPRESENTING HEALTH OF USER

308

GENERATE DATA STRUCTURE REPRESENTING BIOMARKER

310

STORE DATA STRUCTURE IN HARDWARE STORAGE DEVICE

FIG. 3

400

402
OBTAIN PHYSIOLOGICAL DATA
REPRESENTING CARDIAC ACTIVITY
OF USER

404
OBTAIN ACCELERATION DATA
REPRESENTING MOVEMENT OF USER

406
DETERMINE HEARTBEAT INTERBEAT
INTERVALS OF USER

408
DETERMINE MEDIAN OF HEARTBEAT
INTERBEAT INTERVALS OF USER

410
COMBINE MEDIAN OF HEARTBEAT INTERBEAT INTERVALS AND ACCELERATION
DATA

412
GENERATE DATA STRUCTURE REPRESENTING BIOMARKER OF USER

414
STORE DATA STRUCTURE IN HARDWARE STORAGE DEVICE

FIG. 4

FIG. 5

Europäisches
Patentamt

European
Patent Office

Office européen
des brevets

# EUROPEAN SEARCH REPORT

**Application Number**

EP 25 18 9490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/287177 A1 (HUPPERT GILBERT LEE [US] ET AL) 6 October 2016 (2016-10-06) * paragraphs [0047] - [0060], [0100], [0101]; figure 24 * | 1-15 | INV. A61B5/11 |
| X | EP 0 947 160 A1 (SEIKO EPSON CORP [JP]) 6 October 1999 (1999-10-06) * paragraphs [0032] - [0035], [0039] * | 1-15 | |
| A | US 2009/076397 A1 (LIBBUS IMAD [US] ET AL) 19 March 2009 (2009-03-19) * paragraph [0364] * | 6 | |
| A | US 6 882 955 B1 (OHLENBUSCH NORBERT [US] ET AL) 19 April 2005 (2005-04-19) * column 67, line 57 - column 68, line 14 * | 6 | |
| A | US 2024/049982 A1 (COSTA MARIO [SE]) 15 February 2024 (2024-02-15) * paragraph [0090] * | 6 | TECHNICAL FIELDS SEARCHED (IPC) A61B |

The present search report has been drawn up for all claims

| Place of search | Date of completion of the search | Examiner |
|---|---|---|
| Berlin | 24 November 2025 | Trachterna, Morten |

EPO FORM 1503 03.82 (P04C01)

## ANNEX TO THE EUROPEAN SEARCH REPORT
## ON EUROPEAN PATENT APPLICATION NO.

EP 25 18 9490

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2025

| Patent document cited in search report | Publication date | Patent family member(s) | Publication date |
|---|---|---|---|
| US 2016287177 A1 | 06-10-2016 | CA 2930740 A1 | 28-05-2015 |
| | | CN 105813545 A | 27-07-2016 |
| | | EP 3071096 A1 | 28-09-2016 |
| | | JP 6711750 B2 | 17-06-2020 |
| | | JP 7296344 B2 | 22-06-2023 |
| | | JP 2017500093 A | 05-01-2017 |
| | | JP 2020142118 A | 10-09-2020 |
| | | KR 20160088882 A | 26-07-2016 |
| | | US 2016287177 A1 | 06-10-2016 |
| | | US 2018199884 A1 | 19-07-2018 |
| | | WO 2015077559 A1 | 28-05-2015 |
| EP 0947160 A1 | 06-10-1999 | CN 1242693 A | 26-01-2000 |
| | | DE 69833656 T2 | 17-08-2006 |
| | | EP 0947160 A1 | 06-10-1999 |
| | | US 6361501 B1 | 26-03-2002 |
| | | WO 9909884 A1 | 04-03-1999 |
| US 2009076397 A1 | 19-03-2009 | US 2009076397 A1 | 19-03-2009 |
| | | WO 2009036319 A1 | 19-03-2009 |
| US 6882955 B1 | 19-04-2005 | NONE | |
| US 2024049982 A1 | 15-02-2024 | CN 114929104 A | 19-08-2022 |
| | | EP 4247250 A1 | 27-09-2023 |
| | | US 2024049982 A1 | 15-02-2024 |
| | | WO 2022144081 A1 | 07-07-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 77417724